# EUROPEAN PATENT APPLICATION

(11) **EP 3 290 510 A1**
(43) Date of publication of application: **07.03.2018**
(21) Application number: 16001898.2
(22) Date of filing: 31.08.2016
(51) Int. Cl.: C12N 5/00

(54) **MODEL KIT FOR SUSPENSION CELL LINES**

(71) Applicant: Rheinisch-Westfälische Technische Hochschule (RWTH) Aachen, 52056 Aachen (DE)
(72) Inventor: Thalmann, Beat, 69115 Heidelberg (DE); Schiwy, Andreas Herbert, 52064 Aachen (DE); Hollert, Henner, 69493 Hirschberg (DE); Xiao, Hongxia, 52074 Aachen (DE)
(74) Representative: Müller-Boré & Partner Patentanwälte PartG mbB

(57) **Abstract**

The present invention relates to a method for transforming an adherent cell line to a suspension cell line, comprising the following steps of (i) providing one or more adherent cells of an adherent cell line on a culture dish; (ii) dissociating the cells from the culture dish; (iii) transferring the cells to a serum-free and chemically defined medium; and (iv) growing the cells in suspension. Further, the present invention relates to a suspension cell line produced by said method and the use of said suspension cell line for enzyme induction, for manufacturing a defined lysate, for high throughput screening and for tissue and/or spheroid culture.

## Description

The present invention relates to a method for transforming an adherent cell line to a suspension cell line, comprising the following steps of (i) providing one or more adherent cells of an adherent cell line on a culture dish; (ii) dissociating the cells from the culture dish; (iii) transferring the cells to a serum-free and chemically defined medium; and (iv) growing the cells in suspension. Further, the present invention relates to a suspension cell line produced by said method and the use of said suspension cell line for enzyme induction, for manufacturing a defined lysate, for high throughput screening and for tissue and/or spheroid culture.

For the investigation of analytical cell lines, adherent cells are commonly used which adhere to the surface of a cell culture dish. As a consequence, in the process of cell propagation, in order to transfer the adherent cells to a new culture dish, the application of extensive steps for the dissociation of said cells from the dish is required. Moreover, this adherence of the cells is disadvantageous when applying analytical methods on said cells, since only one or two end points can be evaluated for one analyte.

In addition, for the cultivation of analytical cells, fetal calf serum (FCS) is commonly used as a supplement in a cell culture medium. In general, FCS may contain unwanted agents that can contaminate the cell cultures and the biological products obtained therefrom. Further, numerous problems (e.g. the varying quality in composition of the different batches and the risk of contamination with mycoplasma, viruses or prions) exist, particularly if the cells are used for production of drugs or vaccines for human administration. Moreover, the recovery of FCS is not consistent with the goal to reduce animals in testing.

Thus, the problem underlying the present invention is to provide a method for growing a cell line, which does not have the disadvantage of adhering to a surface, in a serum-free and chemically defined medium.

The solution to the above technical problem is achieved by the embodiments characterized in the claims.

In particular, according to a first aspect, the present invention relates to a method for transforming an adherent cell line to a suspension cell line, comprising the following steps:
(i) providing one or more adherent cells of an adherent cell line on a culture dish;
(ii) dissociating the cells from the culture dish;
(iii) transferring the cells to a serum-free and chemically defined medium; and
(iv) growing the cells in suspension.

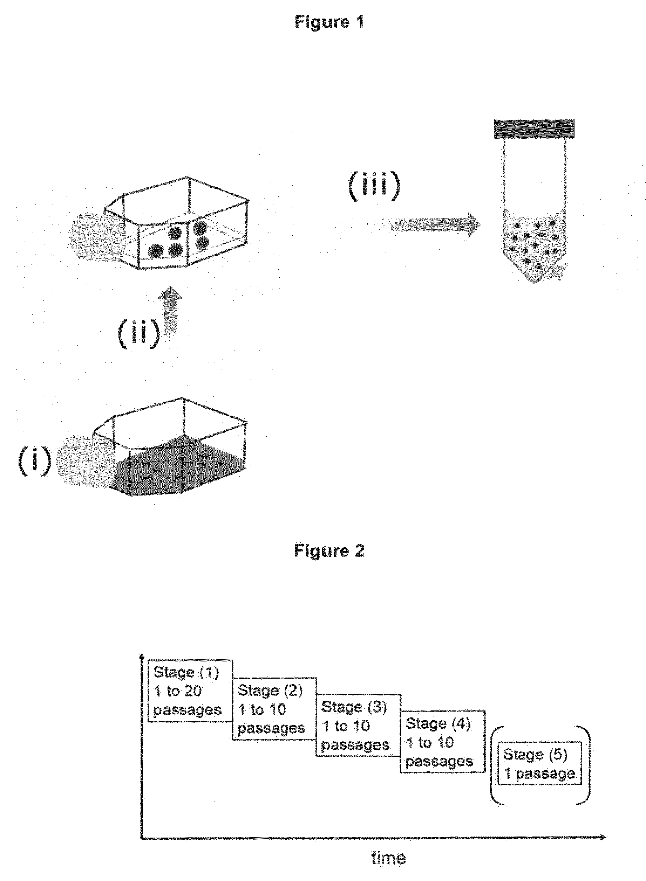

A preferred embodiment of the method according to the present invention is shown in Figure 1.

The term "adherent" in the expression "adherent cell line" means that the cells need a surface on which they may grow. The adherent cell line used in the present invention may be at least one cell line selected from the group consisting of an analytical cell line, a primary cell line or a stem cell line. According to a preferred embodiment of the invention, the adherent cell line is selected from the group consisting of line H4IIE, HepG2, ZFL, RTL-W1, H295R, V79, LNCaP, T47D, U2OS and T47Dkbluc.

The term "analytical cell line" covers cell lines possessing a metabolic function (e.g. organ-specific enzyme production, steroid secretion), a transgenic reporter gene and/or a specific chemically induced morphological behaviour (e.g. micronucleus formation, phenotypic changes, differentiation). This compromises analytics on risk assessment of chemicals and pharmaceuticals, spheroid culture, stem cell differentiation as well as the fate of tumour progression. Analytical cell lines possess the organ-specific, differentiated or a stem cell-like dedifferentiated pheno- as well as genotype. The term "analytical cell line" stands in contrast to the term "production cell line" that compromises cell lines such as CHO-K1, CHO-DG44, HEK293 and Per.C6™, which possess no relevant metabolic activity or analytical capacity and are used for transgenic protein or virus production only.

In the present invention, the expression "suspension cell line" means that the cells of said cell line can be stably cultured in suspension and in a liquid medium.

In a preferred embodiment, the method according to the present invention comprises an additional step between step (i) and (ii). In particular, a step of washing may be performed. The washing medium is not particularly limited and may be phosphate-buffered saline (PBS). According to a preferred embodiment, the PBS is free of calcium and magnesium.

Step (ii) of dissociating the cells from the culture dish is not particularly limited and commonly known methods may be used. For example, the method of dissociation may be selected from the group consisting of adding an effective amount of cell dissociation solution to the cells or mechanically dissociating the cells. In a preferred embodiment according to the present invention, the cell dissociation solution is selected from trypsin or EDTA. Further, the alternative of mechanically dissociating the cells may be employed by using a cell scraper, vibration or a pipette.

The step of dissociation may be performed without removing the cells from the original culture dish. In particular, in said step the cell dissociation solution may be applied to the surface of the adherent cell line of step (i). Then the cell dissociation solution is immediately poured off and the treated cells are incubated for an adapted amount of time, e.g. 5 minutes, under conditions which allows the dissociation of the cells. After the incubation, the cells may be resuspended by e.g. adding a growth supporting medium.

By performing step (ii) of dissociating the cells, the cells are brought into a suspended state. According to a preferred embodiment of step (ii), the cells are brought into a suspended state by constant agitation.

In a preferred embodiment, the method according to the present invention comprises at least one additional step between step (ii) and (iii). In particular, as a first step after step (ii), cell counting may be performed. Then, as further steps, a specific amount of cells may be selected and centrifugation may be applied. In case that the dissociation of step (ii) is performed by using a cell dissociation solution, said solution may be removed by centrifugation.

In step (iii) of the method according to the present invention, the cells are transferred to a serum-free and chemically defined medium.

The expression "chemically defined medium" used in the present invention means that all chemical components of said medium are known. In addition, the wording "serum-free" specifying the chemically defined medium of the present invention refers to the absence of any animal-derived serum, particularly of fetal calf serum, in the medium. The expression "chemically defined" specifies the absence of complex biological mixtures such as tryptic peptone from plant, yeast or animal source, undefined peptide and/or lipid mixtures.

The serum-free and chemically defined medium used in the method of the present invention is not particularly limited and may be based on any basal medium such as DMEM, Ham's F12, Medium 199, McCoy or RPMI generally known to the skilled worker. The basal medium may comprise a number of ingredients, including amino acids, vitamins, organic and inorganic salts, and sources of carbohydrate, each ingredient being present in an amount which supports the cultivation of a cell which is generally known to the person skilled in the art. The medium may contain auxiliary substances, such as buffer substances like sodium bicarbonate and/or HEPES, antioxidants, stabilisers to counteract mechanical stress, or protease inhibitors. If required, a non-ionic surfactant such as mixtures of polyethylene glycols and polypropylene glycols (e.g. Pluronic F68 ®, SERVA) can be added as a defoaming agent.

According to a preferred embodiment, the serum-free and chemically defined medium comprises at least one compound selected from a cell line-specific supplement, a serum-substituting supplement and a suspension-stabilizing compound.

The cell line-specific supplement is not particularly limited and may be chosen depending on the cell line used in the present invention. For example, the cell line-specific supplement may be selected from at least one compound of the group consisting of serum-derived or recombinant albumin such as e.g. human or bovine serum albumin, insulin, epidermal growth factor (EGF; rhEGF: recombinant human EGF), or 4-(2-hydroxyethyl)-1-piperazineethanesulfonic acid (HEPES). An exemplary list of eventual specific supplements is shown at Table 1. The cell lines of Table 1, for which no specific supplement is listed, can be supplemented with any of the above specific supplements alone or in combination.

**Table 1: Exemplary suspension cell lines and their specific supplements.**

| Cell line | Specific supplement |
|---|---|
| H295R-S | rhEGF |
| H4IIE-S | - |
| HepG2-S | - |
| LNCaP-S | - |
| RTL-W1-S | Insulin, HEPES |
| T47D-S | rhEGF, Insulin |
| T47Dkbluc-S | rhEGF, Insulin, G418 |
| U2OS-S | - |
| V79-S | - |
| ZFL-S | rhEGF, HEPES |

The serum-substituting supplement used in the present invention is not particularly limited and may be chosen from supplements commonly known to the skilled person. The serum-substituting supplement may be at least one compound of the group consisting of CTS™ B-27® Supplement, XenoFree (ThermoFisher), human platelet lysate (hPL) (by e.g. ThermoFisher or PL Bioscience), insulin-transferrin-selenium (ITS) (e.g. by ThermoFisher), Panexin NTS (PAN Biotech) and Xcyte™ and Xcyte Plus™ Xeno-Free Supplement (iBiologics).

The suspension-stabilizing compound of the present invention is not particularly limited as long as the compound provides a suspension-stabilizing effect to the suspended cells. According to a preferred embodiment of the present invention, the suspension-stabilizing compound is at least one compound selected from polyethylene glycol (PEG) and a peptide mimicking an extracellular matrix protein. According to a preferred embodiment, extracellular matrix proteins and their specific peptide mimicking an extracellular matrix protein such as a RGD-type peptide are used with or without PEG to initially stabilise the growth in suspension.

Polyethylene glycol can act as an extracellular matrix and helps preventing the aggregation of the suspended cells. According to a preferred embodiment of the present invention, PEG has a molecular weight from 5000 to 50000 g/mol, more preferred from 7000 to 10000 g/mol and most preferred of 8000 g/mol. When PEG having a molecular weight of 8000 g/mol (i.e. PEG8000, Sigma, #89510) is used, the viability of the cells and the viscosity of the serum-free and chemically defined medium can be improved. The molecular weight of PEG used in the present invention represents the value given by the respective manufacturer (e.g. Sigma-Aldrich Chemise GmbH, Munich, Germany).

The peptide mimicking an extracellular matrix protein can reduce the aggregation of cells. An extracellular matrix can provide structural and biochemical support to the surrounding cells. In the present invention, the peptide is not particularly limited. In a preferred embodiment of the present invention, the peptide is a RGD-type peptide which is an integrin-binding peptide. The term "RGD" refers to the sequence "Arg-Gly-Asp". According to a preferred embodiment of the present invention, the general sequence of the peptide is (N)₀₋₁RGD(N)₀₋₆, wherein "N" represents one of the standard amino acids. Specifically, the peptide can be selected from the group consisting of RGDS (Arg-Gly-Asp-Ser) according to SEQ ID NO: 1, RGDC (Arg-Gly-Asp-Cys) according to SEQ ID NO: 2, RGDF (Arg-Gly-Asp-Phe) according to SEQ ID NO: 3, CRGDSPASSC (Cys-Arg-Gly-Asp-Ser-Pro-Ala-Ser-Ser-Cys) according to SEQ ID NO: 4, RGDSPASSKP (Arg-Gly-Asp-Ser-Pro-Ala-Ser-Ser-Lys-Pro) according to SEQ ID NO: 5 and GRGDSPK (Gly-Arg-Gly-Asp-Ser-Pro-Lys) according to SEQ ID NO: 6.

In a preferred embodiment, the method according to the present invention comprises an additional step between step (iii) and (iv), wherein the composition of the serum-free and chemically defined medium is changed in a multitude of number of passages. By applying the multitude of number of passages, the adaptation of the cells to the serum-free and chemically defined medium and the stabilization in suspension can be improved.

In the present invention, the wording "adaptation" of a cell denotes changes made by a cell due to environmental changes, i.e. for example due to a change of the culture medium.

The term "passage" used herein refers to subculturing cells, wherein some or all cells from a previous culture are transferred to a fresh growth medium.

According to a preferred embodiment of the present invention, the amount of the serum-substituting supplement in the serum-free and chemically defined medium decreases with the increasing number of passages. By providing the serum-substituting supplement at the beginning of the multitude of number of passages, the initial surviving rate of the cells can be increased by avoiding serum deprivation. At a higher number of passages it is preferred to reduce the amount of the serum-substituting supplement because of its in later stages obsolete role or the cell line-depending inhibitory effects (e.g. T47D). In a preferred embodiment of the present invention, the serum-free and chemically defined medium does not comprise a serum-substituting supplement in the last passage.

In a preferred embodiment of the present invention, the amount of polyethylene glycol in the serum-free and chemically defined medium decreases with the increasing number of passages. By providing PEG at the beginning of the multitude of number of passages, the cell suspension can be stabilized by the extracellular matrix formed of PEG. At a higher number of passages, it is preferred to reduce the amount of PEG such that a PEG-independent suspension cell line can be obtained.

According to a preferred embodiment of the present invention, the amount of the peptide mimicking an extracellular matrix protein in the serum-free and chemically defined medium increases with the increasing number of passages. By using the peptide mimicking an extracellular matrix protein, the cell suspension can be stabilized. In addition, the presence of the peptide mimicking an extracellular matrix protein in the serum-free and chemically defined medium supports the selection towards the growth of a single cell line. However, said peptide has the disadvantageous effect of hindering cell growth in a cell line-dependent manner. Accordingly, in a preferred embodiment, the peptide mimicking an extracellular matrix protein is present in the serum-free and chemically defined medium only for 1 to 20, more preferred for 2 to 10, passages.

In a preferred embodiment of the present invention, the cell line-specific supplement is used in all of the multitude of number of passages.

According to a preferred embodiment of the present invention, the multitude of number of passages is divided into a multitude of stages. A preferred embodiment of the method of the present invention comprising stages (1) to (4) and an optional stage (5) as described below is shown in Figure 2. Preferably, in all stages (1) to (4) and in optional stage (5) the serum-free and chemically defined medium comprises the cell line-specific supplement.

Preferably, the multitude of stages comprises:
Stage (1): wherein the serum-free and chemically defined medium comprises the serum-substituting supplement in an amount of 1 to 10 wt% and PEG in an amount of 0.5 to 10 wt%. According to a preferred embodiment, the conditions of stage (1) are met during 1 to 20 passages, preferably during 2 to 18 passages and more preferred during 3 to 15 passages.
Stage (2): wherein the serum-free and chemically defined medium comprises the serum-substituting supplement in an amount of 1 to 3 wt%, more preferred 1 wt%, and PEG in an amount of 0.5 to 1 wt%. According to a preferred embodiment, the conditions of stage (2) are met during 1 to 10 passages, preferably during 1 to 8 passages and more preferred during 1 to 5 passages.
Stage (3): wherein the serum-free and chemically defined medium does not comprise the serum-substituting supplement, but does comprise 0.5 to 1 wt% PEG and 0.5 to 5 mg/L of the peptide mimicking an extracellular matrix protein. According to a preferred embodiment, the conditions of stage (3) are met during 1 to 10 passages, preferably during 1 to 8 passages and more preferred during 1 to 5 passages.
Stage (4): wherein the serum-free and chemically defined medium does not comprise the serum-substituting supplement and PEG, but does comprise the peptide mimicking an extracellular matrix protein in an amount of 0.5 to 5 mg/L, more preferred of 0.5 to 1 mg/L. According to a preferred embodiment, the conditions of stage (4) are met during 1 to 10 passages, preferably during 1 to 8 passages and more preferred during 1 to 5 passages.

By using the conditions defined in the above stages (1) to (4), the adaptation of the cells to the serum-free and chemically defined medium can be facilitated and a stable growth of the cells in suspension in accordance with step (iv) of the invention can be achieved.

In the present invention, as an additional optional stage (5), the serum-free and chemically defined medium does not comprise the serum-substituting supplement, PEG and the peptide mimicking an extracellular matrix protein. According to a preferred embodiment of the present invention, the conditions of stage (5) are met in one passage, i.e. the last passage of the method.

After performing the passage of stage (5), it is preferably evaluated whether the composition of the cell line-specific supplement should be changed, reduced or omitted. By adapting the composition of the cell line-specific supplement, i.e. by for example adding a specific type of salt and/or growth factors, cell line-specific characteristics or the growth of the cells can be influenced. For example, by adjusting the composition of the cell line-specific supplement, spheroids can be formed by the suspended cells.

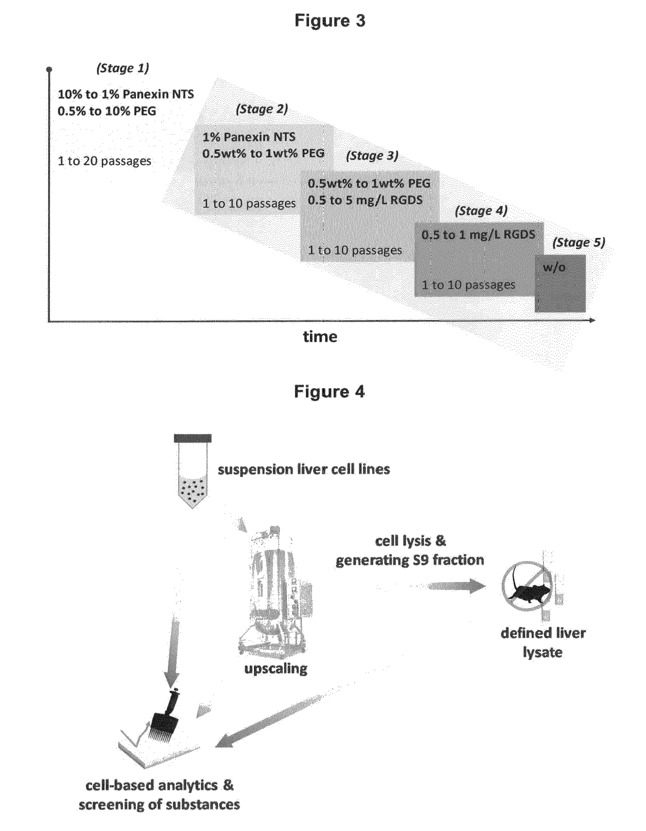

In Figure 3, a specific embodiment of the present invention is shown, wherein the serum-substituting supplement is Panexin NTS and the peptide mimicking an extracellular matrix protein is RGDS. The wording "w/o" in stage (5) means that this passage is performed without the use of the serum-substituting supplement, PEG and the peptide mimicking an extracellular matrix protein.

In step (iv) of the method of the present invention, the cells are grown in suspension. In particular, the cells can be grown in a continuous suspension. The term "continuous suspension" means that the cells grow stably in suspension. As a result, a suspension cell line can be obtained. In a preferred embodiment of the present invention, growing the cells in suspension is performed after the last passage, i.e. the last passage of stage (4) or the passage of stage (5). According to a preferred embodiment, in step (iv), the cell line-specific supplement is changed in accordance with the conditions outlined with respect to stage (5) above.

According to a second aspect, the present invention relates to a suspension cell line produced by the above-described method. The suspension cell line according to the invention is stable in growth (e.g. growth rate, viability cell density), morphology (e.g. cell size) and response (e.g. metabolic enzyme production upon xenobiotic stimulus and/or reporter gene induction). The derived suspension cell lines are able to be grown in shaken vessels such as filter tubes, single-use bioreactors and/or stirred tank vessels for bulk cell production. As a consequence, the suspension cell line of the invention can be converted to intact spheroids by partially changing the medium composition (e.g. by Ca²⁺, stimulating peptides and/or growth factors).

According to a third aspect, the invention relates to the use of the suspension cell line of the present invention for enzyme induction. This means that at least one compound can be used on said suspension cell line in order to induce the expression of an enzyme. In a preferred embodiment of the present invention, the compound used for enzyme induction is at least one selected from the group consisting of butyric acid, valeric acid, isovaleric acid, valproic acid and salts thereof.

In addition, according to a fourth aspect, the invention relates to the use of the suspension cell line of the present invention for manufacturing a defined lysate. The suspension cell line of the present invention can be directly applied in analytical test methods. Based on standardized pre-incubation (i.e. enzyme induction) and the addition of lysis, lysates from the suspension cell line can be produced. Preferably, the enzyme induction is performed by the previously defined use of the suspension cell line for enzyme induction. According to a preferred embodiment of the present invention, suspension liver cell lines are used which can be turned into qualitatively consistent S9 fractions as indicated in Figure 4. Said fractions can be employed in *in situ* metabolism. Commonly used S9 fractions are produced by using rat or human liver, wherein in the former case, the enzyme induction is performed directly in the animal. By using the present invention instead, a defined liver lysate results which is favorable from the ethical viewpoint and which allows an efficient scaie-up of the production.

According to a fifth aspect, the invention relates to the use of the above-described suspension cell line for high throughput screening.

The present invention allows the adaptation of toxicologically relevant analytical adherent cell lines, primary cell lines and/or stem cells to suspension cell culture in a serum-free and chemically defined medium. Moreover, the present invention enables analytical cell lines to continuously grow in suspension without the change in differential state. This leads to a higher robustness of the test system and a reduction of the variability within the system. In addition, by growing the cells in suspension in accordance with the present invention, a higher cell density in contrary to systems employing adherent cells can be achieved. Compared with commonly used techniques, the present invention improves high throughput application, sustainability, efficiency and quality in analytics. By employing the present invention, it is possible to achieve independency of the costly compound serum and an up to now unequalled inexpensive high throughput application for analytical cell lines. In particular, the present invention provides a fast establishment of serum-free suspension cell lines.

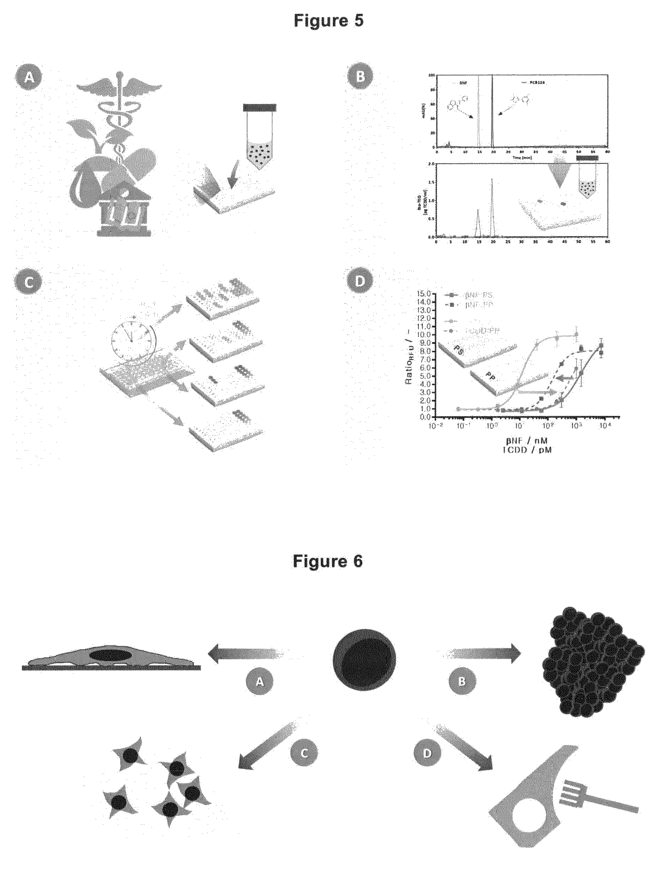

Due to the present invention, experimental efficiency and flexibility can be improved as shown in Figure 5:

Since the cell density of the cell suspension can be easily determined in a non-invasive manner, the cell suspension resulting from the method can be directly applied to a test plate which has already been prepared with dissolved or solid substances of pharmaceutical or toxicological relevance of synthetic or biological origin as indicated in Figure 5A. Therefore, the present invention can be employed in high-throughput screening of complex ecological samples and substance libraries and facilitates the combination of chemical and cell-based analytics. As shown in Figure 5B, HPLC fractions of complex substance mixtures based on ecological samples or syntheses or established substance libraries can be directly analyzed by using the suspension cell line of the present invention in a high-throughput method without the addition of a solvent. Within one day it is possible to draw a conclusion concerning the toxicity, effects and chemical structure of a sample. The high throughput-based analytical method can be ideally automated by using a suitable pipetting robot, wherein the bioassay is handled semi-sterilely by a limited incubation time.

Further, as indicated in Figure 5C, the present invention allows acceleration and multiplication of cell-based test systems: After incubation with an analyte, the sample can be distributed to a multitude of plates for a multiple end point determination. This is possible due to the fact that the cells produced by the present invention stay in a suspended state. On the contrary, for adherent cell lines the above described acceleration and multiplication is not possible.

In addition, as shown in Figure 5D, the suspension cell culture of the present invention allows the application of a variety of plate materials. Because of this flexible choice of the plate material, it is possible to analyze substances which usually adhere to the plate material in commonly used methods and therefore are not able to enter the cells. By using e.g. polypropylene (PP) as a plate material, which is not possible in the case of using adherent cell lines, the sensitivity regarding hydrophilic substances with a hydrophobicity comparable to those of pharmaceutical substances (e.g. beta-naphtoflavone), can be increased. Therefore, the application of the present invention enables a flexibilization of the experimental designs and an early detection of possible effects of pharmaceutically relevant substance groups.

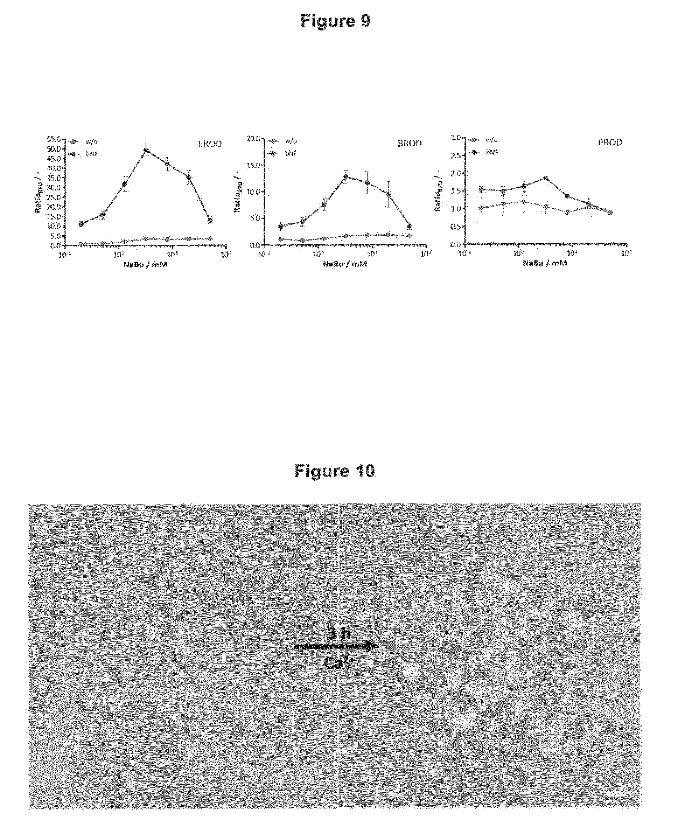

The present invention covers the adaptation of adherent cell lines, stem cell and tissues to metabolically intact and stable suspension cell lines for analytical purposes, tissue engineering as well as regenerative medicine and/or *in vitro* meat production. The outlined procedure towards a stable single-cell suspension culture maintains the differential state of a formerly adherent cell line or tissue. This enables the direct use of subsequent suspension cell lines for e.g. high throughput screening of pharmaceutical substance libraries, single substances, complex environmental samples (Figure 5A) and chromatographic fractions (Figure 5B). After incubation, the treated cell suspension can be splitted into different vessels for multiple endpoint determination (Figure 5C). Furthermore, the established suspension cell lines can be incubated and cultured on different materials to reach the optimal assay sensitivity (Figure 5D). The suspension cell lines of the present invention can be upscaled in an efficient way to increase the needed high cell density for subsequent purposes of analytical manner or for use in e.g. regenerative medicine. In this very special application, suspension liver cell lines (H4IIE-S, HepG2-S, ZFL-S or directly tissue-derived cell lines) can be used for large-scale, ethical S9 fraction lysate production for replacing the rat or human liver derived S9 fractions (Figure 4). By using butyric acid, valeric acid, isovaleric acid or valproic acid including their salts, the enzyme activity and efficiency of either the suspension cell lysate or whole cell suspension is increased. In a more general embodiment, suspension cell lines with stable differential state can be efficiently converted to either adherent monolayer cultures, three-dimensional spheroid cultures, matrix embedded tissue cultures and/or *in vitro* meat production by applying specific factors (e.g. growth hormones, ions) (Figure 6; Figure 10).

The figures show:
Figure 1: A preferred embodiment of the method according to the present invention. In step (i), one or more adherent cells of an adherent cell line are provided on a culture dish; in step (ii), the cells are dissociated from the culture dish; in step (iii), the cells are transferred to a serum-free and chemically defined medium; and are finally grown in suspension.
Figure 2: Preferred embodiment of the present invention, wherein the method comprises an additional step between step (iii) and (iv), wherein the composition of the serum-free and chemically defined medium is changed in a multitude of number of passages. In particular, in the preferred embodiment, the multitude of number of passages is divided into stages (1) to (4) and an optional stage (5).
Figure 3: Specific embodiment of the present invention, wherein in the method the composition of the serum-free and chemically defined medium is changed in five stages.
Figure 4: Examples of advantageous effects of the present invention, especially regarding the production of upscaling and the production of a defined cell lysate for *in vitro* metabolisation.
Figure 5: An application example of a suspension cell line resulting from the present invention.
Figure 6: Examples of advantageous effects of the present invention, especially regarding the conversion of the present suspension cell lines in adherent cell lines (A), spheroid cell cultures (B), tissue culture (C) as well as exemplary the application towards artificial *in vitro* meat for consumption (D).
Figure 7: Adaptation of the adherent reporter cell line T47Dkbluc. (a): passaging has been performed using insulin and Panexin NTS; (b): in addition to (a), PEG8000 was used; and (c): Compared to (b), also RGDS was used during passaging.
Figure 8A: Influence of RGDS and Panexin NTS on T47Dkbluc-S in a concentration-dependent manner.
Figure 8B: Influence of Panexin NTS on ZFL-S including RGDS in a concentration-dependent manner.
Figure 8C: The suspension cell line HepG2-S is suitable for the detection of substances which have a similar effect like dioxin (in the present case: TCDD). RGDS does not have any effect on the CYP1A1 induction by TCDD.
Figure 9: Increased induction of the CYP enzyme activity by using sodium butyrate (NaBu) in suspension cell line H4IIE-S (rat): By employing NaBu, the yield of phase I and phase II enzymes can be quantitatively improved (not shown). Due to the adjustment to the optimal concentration of NaBu, a multitude of biotransformation enzymes can be upregulated. bNF: beta-naphthofiavone; EROD: ethoxyresorufin-O-dealkylase; BROD: butyloxyresorufin-O-dealkylase; PROD: pentyloxyresorufin-O-dealkylase.
Figure 10: Induced aggregation of suspension cell line H4IIE-S (rat) towards spheroid formation using increased Ca²⁺ concentration: By employing the 5-fold concentration of Ca²⁺, the cell-cell aggregation could be induced within three hours enabling spheroid cell culture.

The sequences are as follows:
SEQ ID NO: 1
   Arg Gly Asp Ser
SEQ ID NO: 2
   Arg Gly Asp Cys
SEQ ID NO: 3
   Arg Gly Asp Phe
SEQ ID NO: 4
   Cys Arg Gly Asp Ser Pro Ala Ser Ser Cys
SEQ ID NO: 5
   Arg Gly Asp Ser Pro Ala Ser Ser Lys Pro
SEQ ID NO: 6
   Gly Arg Gly Asp Ser Pro Lys

The present invention will now be further illustrated in the following examples without being limited thereto.

### Examples

### Methods

### Adherent cell culture methods

Previously to the adaptation, adherent cell lines were cultured in 25 cm², 75 cm² or 125 cm² cell culture flasks (TPP, Transadingen, Switzerland; Greiner, Frickenhausen, Germany) and/or 6-, 12-, 24- or 96-well plates (TPP, Transadingen, Switzerland; Greiner, Frickenhausen, Germany) using DMEM, RPMI1641, DMEM/F12, L15 and/ or MEM basal media (100 µl - 20 ml; Gibco, Darmstadt, Germany) with the respective essential supplements FCS (1 - 20%; e.g. Biowest Europe, Nuaillé, France) or hPL (1 - 10 %; e.g. PL Bioscience, Aachen, Germany) or Panexin NTA (1 - 10 %; e.g. PAN-Biotech, Aidenbach, Germany) and/or ITS or ITS+ (1x; BD Biosciences, Heidelberg, Germany). Cell line dependent, the culture media were supplemented with e.g. MEM NEAA, Glutamax or L-Glutamin, bovine or recombinant human Insulin, recombinant human EGF, recombinant human Transferrin and/or HEPES (Gibco, Darmstadt, Germany; Sigma-Aldrich Chemie GmbH, Munich, Germany).

The standard cultivation settings are, unless otherwise mentioned: 4.5-5.0 % CO₂, 36.8 ± 0.5°C, >90% rel. humidity. For the fish cell lines following cultivation settings are: 19.8 ± 0.5°C (RTL-W1) or 27.8 ± 0.5°C (ZFL), >90% rel. humidity. For passaging, the cells were trypsinated. Cell counts can be estimated by Neubauer haemocytometer (Brand, Wetheim, Germany) with or without prior trypsination with Trypsin 0.5%/EDTA (Gibco, Darmstadt, Germany) and erythrosine B (Roth, Karlsruhe Germany) or Trypan Blue (Gibco, Darmstadt, Germany) staining or counted by instrumental cell counters (Coulter Counter from Beckman, Krefeld, Germany; CASY, Cedex or Cellavista from Innovatis, Roche, Mannheim, Germany).

### General suspension cell culture methods

The standard batch suspension cell cultures can be performed in TubeSpin Bioreactor 50 (TPP, Transadingen, Switzerland) using the medium CDM4PerMAb (HyClone, Thermo, Logan, Utah) supplemented with 5 mM Glutamax (Gibco, Darmstadt, Germany) or another chemically defined, serum-free medium suitable for cultivating suspension cell lines. The standard cultivation settings are, unless otherwise mentioned: 5-15 ml Medium, 220 rpm, Shaker amplitude (e.g. DOS-20M, LTF Labortechnik GmbH & Co. KG, Wasserburg, Germany) of 20 mm, 4.5-5.0 % CO₂, 36.8 ± 0.5°C, Airation (BFT50): A-E. For fish suspension cell lines the following temperatures are applied: 19.8 ± 0.5°C (RTL-W1-S) or 27.8 ± 0.5°C (ZFL-S).

Cell counts can be estimated using the methodical description above.

### Assaying

For assays for determining reporter gene or enzyme activities, the desired cell line was incubated with different concentrations of receptor agonists (e.g. estradiol, dexamethasone, β-naphtoflavone), antagonists and/or metabolic shift inducers such as sodium butyrate, valproic acid or valeric acid and the respective media in 96-well or 384-well plates (TPP, Transadingen, Switzerland; Greiner, Frickenhausen, Germany). Reporter protein as well as enzyme activities were determined by specific substrate conversion leading to a shift in light or fluorescence and/or colour (absorbance) (e.g. D-luciferin, coelenterazine, 7-resorufin esters/ethers) or by direct/indirect ELISA measurement of cellular products.

### Adaptation of the adherent cell lines to suspension

The transition from permanently adherent cell lines or tissues to permanent suspension cell cultures in a chemically defined, serum-free medium is performed by the following general procedure:
1) An adherent cell culture in standard serum-containing medium (Figure 1 i) is recovered in suspension by trypsination (Figure 1 ii). In the following, the present suspension is centrifuged (209·g, room temperature, five minutes) and directly resuspended in a chemically defined, serum-free medium containing initially 10% Panexin NTS (PAN-Biotech, Aidenbach, Germany), 0.5% -10% PEG8000 (Sigma-Aldrich Chemie GmbH, Munich, Germany) and the cell specific supplements (Table 1; Gibco, Darmstadt, Germany) (Figure 1 iii).
2) The following steps include the successive reduction of Panexin NTS as well as PEG8000 and the parallel increase in RGDS (Sigma-Aldrich Chemie GmbH, Munich, Germany) concentration (Figure 2, Stages 1-4). Panexin NTS is initially introduced at 10% and rapidly reduced to 0% (Figure 3, Stages 1 and 2). PEG8000 is introduced for avoiding cell aggregation, which inhibit proper oxygen transfer as well as growth. The latter is then reduced towards full adaptation to single-cell suspension (Figure 3, Stages 1-3). In parallel, the integrin-binding peptide RGDS is introduced (Figure 3, Stages 3 and 4) until stable single-cell suspension culture.
3) Reaching the last step (Figure 3, Stage 5) a stable single-cell suspension culture is established and ready for further passaging without RGDS.

### Results

### Exemplary suspension cell line adaption and the influence of PEG and peptides mimicking an extracellular matrix protein

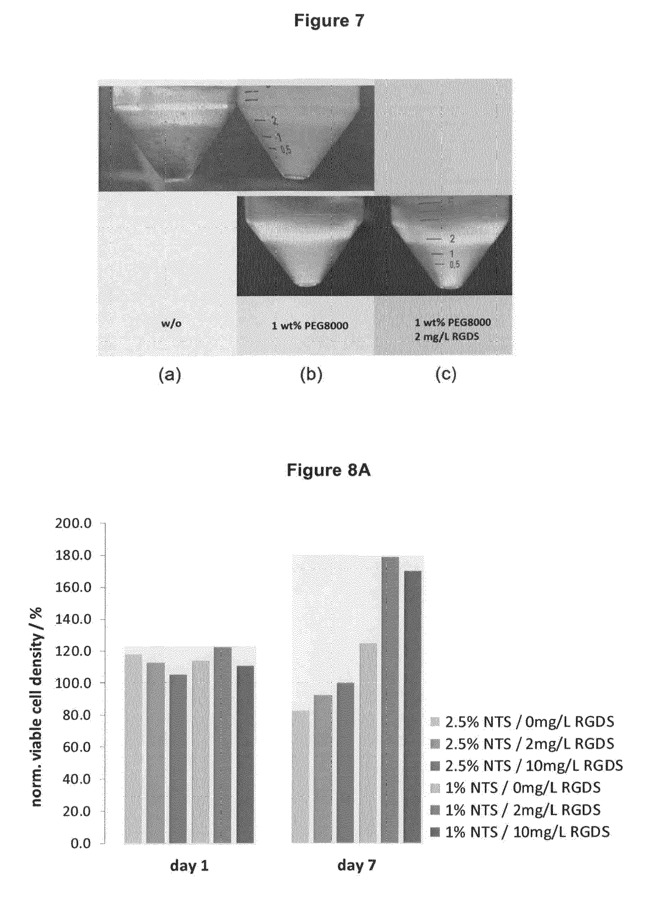

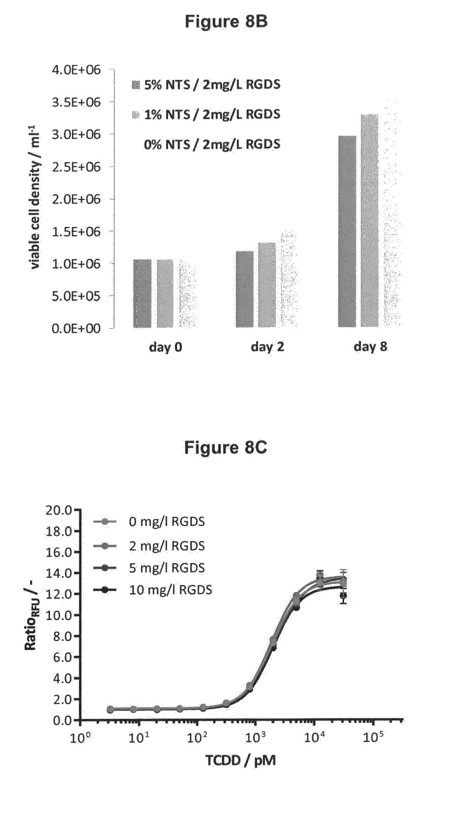

The recombinant reporter cell line T47Dkbluc has been subjected to adaptation in serum-free and chemically defined media (CDM) during 12 passages in total. The results are shown in Figure 7. Here, the subsequent cell line in CDM including 2.5 % Panexin NTS resulted in extended cell aggregation, whereas the parallel addition of 1 wt% PEG (i.e. PEG8000) reversed the effect (six passages) (Figure 7(a) and 7(b)). By subsequent passaging, the reduction to 1 % Panexin NTS and addition of 2 mg/L RGDS, the cell aggregation could be further reduced towards a single-cell suspension (three passages) (Figure 7(b) and 7(c)). The addition of RGDS further improved the growth rate of the established T47Dkbluc-S in a concentration dependent manner, whereas increased Panexin NTS concentrations inhibited the cell growth at the later stage of adaptation (three passages) (Figure 8A). This effect was partially reversed by the increasing concentration of RGDS, whereby the optimal concentration for T47Dkbluc-S was 2 mg/L. Applying RGDS on ZFL-S showed the same growth promoting as well as deaggregating effect (data not shown). In ZFL-S, the reduction of Panexin NTS at later stages of the adaption was crucial as well (Figure 8B). Whilst RGDS showed a significant influence in growth and cell aggregation, the cell specific metabolic or differential state was not influenced, even in feasibly high concentrations. The respective data for the suspension hepatocarcinoma cell line HepG2-S showed that up to 10 mg/L RGDS had no significant impact on TCDD-mediated CYP1A1 induction (measured by 7-Ethoxyresorufin conversion) (Figure 8C).

### Metabolic boost by HDAC inhibitors such as butyric acid, valeric acid, isovaleric acid, valproic acid and salts thereof

The suspension rat hepatoma cell line H4IIE-S was adapted according Figure 3 and was chosen as a model for large-scale S9 fraction lysate production. For improved metabolic enzyme production, the HDAC (histone deacetylase) inhibitor sodium butyrate (NaBu) was applied on H4IIE-S in a concentration-dependent manner (Figure 9). The essential biotransformation phase I enzymes of the cytochrome P450 family are efficiently induced by xenobiotics such as TCDD and/or beta-Naphthoflavone (bNF). Here, the combination of bNF and NaBu resulted in a synergic induction of Cyp1a1/2, Cyp3a4 and partially Cyp2B. Other biotransformation phase I and II enzymes are efficiently induced as well (latter data not shown). Contrary, higher concentrations of NaBu than 3 mM let to a decline in metabolic enzyme production (Figure 9).

### Induction of cell-cell aggregation and spheroid culture derived from H4IIE-S suspension cell line

The previously described suspension rat hepatoma cell line H4IIE-S were treated with the 5-fold Ca²⁺ concentration based on the standard serum-free, chemically defined medium. After three hours, extended spheroidal structures were formed (Figure 10). Prolonged incubation result bigger clusters (data not shown).

## Claims

1. A method for transforming an adherent cell line to a suspension cell line, comprising the following steps:
(i) providing one or more adherent cells of an adherent cell line on a culture dish;
(ii) dissociating the cells from the culture dish;
(iii) transferring the cells to a serum-free and chemically defined medium; and
(iv) growing the cells in suspension.

2. The method according to claim 1, wherein the serum-free and chemically defined medium comprises polyethylene glycol.

3. The method according to claim 2, wherein polyethylene glycol has a molecular weight from 5000 to 50000 g/mol.

4. The method according to any one of claims 1 to 3, wherein a peptide mimicking an extracellular matrix protein is contained in the serum-free and chemically defined medium.

5. The method according to any one of claims 1 to 4, wherein the method comprises an additional step between step (iii) and (iv), wherein the composition of the serum-free and chemically defined medium is changed in a multitude of number of passages.

6. The method according to claim 5, wherein the amount of polyethylene glycol in the serum-free and chemically defined medium decreases with the increasing number of passages.

7. The method according to claim 5 or 6, wherein the amount of the peptide mimicking an extracellular matrix protein in the serum-free and chemically defined medium increases with the increasing number of passages.

8. The method according to any one of claims 5 to 7, wherein the serum-free and chemically defined medium comprises a serum-substituting supplement, the amount of which in the serum-free and chemically defined medium decreases with the increasing number of passages.

9. The method according to any one of claims 4 to 8, wherein the peptide mimicking an extracellular matrix protein is a RGD-type peptide.

10. The method according to claim 9, wherein the peptide mimicking an extracellular matrix protein is RGDS.

11. A suspension cell line produced by the method according to any one of claims 1 to 10.

12. Use of the suspension cell line according to claim 11 for enzyme induction.

13. Use of the suspension cell line according to claim 11 for manufacturing a defined lysate.

14. Use of butyric acid, valeric acid, isovaleric acid, valproic acid and salts thereof, and the suspension cell line according to claim 11 for induction of intrinsic or transgenic protein expression and manufacturing a defined lysate.

15. Use of the suspension cell line according to claim 11 for high throughput screening, or for the induction of tissue as well as spheroid cultures.


SEQUENCE LISTING

<110> RWTH Aachen

<120> Model kit for suspension cell lines

<130> R 2776EU

<160> 6

<170> PatentIn version 3.1

<210> 1
<211> 4
<212> PRT
<213> Artificial

<400> 1

Arg Gly Asp Ser
1

<210> 2
<211> 4
<212> PRT
<213> Artificial

<400> 2

Arg Gly Asp Cys
1

<210> 3
<211> 4
<212> PRT
<213> Artificial

<400> 3

Arg Gly Asp Phe
1

<210> 4
<211> 10
<212> PRT
<213> Artificial

<400> 4

Cys Arg Gly Asp Ser Pro Ala Ser Ser Cys
1               5                   10

<210> 5
<211> 10
<212> PRT
<213> Artificial

<400> 5

Arg Gly Asp Ser Pro Ala Ser Ser Lys Pro
1               5                   10

<210>  6
<211>  7
<212>  PRT
<213>  Artificial

<400>  6

Gly Arg Gly Asp Ser Pro Lys
1               5